# EUROPEAN PATENT SPECIFICATION

(11) **EP 2 483 018 B1**
(45) Date of publication and mention of the grant of the patent: **22.07.2015**
(21) Application number: 10782387.4
(22) Date of filing: 29.09.2010
(51) Int. Cl.: B22F 3/10, B22F 3/11, B22F 5/10, B22F 7/00, A61F 2/00

(54) **METHOD FOR THE REALIZATION OF BIOLOGICALLY COMPATIBLE PROSTHESIS**
VERFAHREN ZUR REALISIERUNG BIOLOGISCH KOMPATIBLER PROTHESEN
PROCÉDÉ DE FABRICATION D'UNE PROTHÈSE BIOCOMPATIBLE

(30) Priority: 30.09.2009 IT VR20090154
(43) Date of publication of application: 08.08.2012
(73) Proprietor: Eurocoating S.p.A., 38057 Trento (IT)
(72) Inventor: BUCCIOTTI, Francesco, I-43040 Solignano (Parma) (IT); ROBOTTI, Pierfrancesco, I-43040 Solignano (Parma) (IT)
(74) Representative: Feltrinelli, Secondo Andrea
(86) International application number: PCT/IB2010/054401
(87) International publication number: WO 2011/039716

(56) References cited:
- WO-A1-2005/072785
- US-A- 4 612 160
- US-A- 6 022 509
- US-A1- 2006 015 187
- US-A1- 2006 157 543

## Description

### TECHNICAL FIELD OF THE INVENTION

The invention regards a method for the realization of biologically compatible prosthesis, particularly suitable for obtaining elements implantable in human and animal body.

### STATE OF THE PRIOR ART

Prosthesis, such as for example acetabular cups, femoral and tibial implants, glenoids and other components of the like, are used for implants into human or animal body so as to replace bone parts or joints which have been irremediably damaged by trauma or diseases such as arthritis and arthrosis.

There are various production techniques. It could be reminded machining for removing chips, casting, Selective Laser Sintering (SLS) and Electron Beam Melting (EBM). The latter two techniques are used to produce prosthesis through deposition and sintering of successive layers of powder.

Some of the external surfaces of the prosthesis are then usually treated to confer porosity and/or roughness features thereto, so as to facilitate or accelerate the osteointegration process.

A particular method, of the subtractive type, is described in the patent application WO2009/004444. A given amount of powder material containing a second removable granular material (also called spacer) having a melting temperature that is higher than the sintering temperature of the former, is placed in mould. The two materials are first mixed homogeneously, and subsequently subjected to a sintering and solidification process. The obtained object, concave and/or convex, is submerged in water or in an acid or basic solution. Submersion into the solution allows dissolving the spacer material, which leaves the volume it had occupied in the pressing and sintering step empty. After completing dissolution, the piece, which is made up of only the first material, is characterized by the presence of pits and pores on the surface.

This method has limited degrees of freedom regarding the realization of the prosthesis, especially due to the fact that the porous surface and the layer on which it lies are made of the same material. However - consider an acetabulum for example - the ideal choice would be that of having a porous surface made up of a biocompatible material with high osteointegration capacity on an underlying base with high mechanical resistance and resistant to wear. Systems with these two characteristics are not frequent and however do not entirely meet the aforementioned characteristics.

The patent application No. US 2006/0015187 discloses a porous medical implant made by bonding or simultaneously creating and bonding a porous structure to a solid substrate using pulsed electrical sintering; the porous surface is formed by application of pulsed electrical energy in such a way to cause a localized heating in the surface of the material comprising portions of the implant

### OBJECTS OF THE INVENTION

The main object of the invention is defining a method for the realization of biologically compatible prosthesis having greater degrees of freedom in the realization of the prosthesis themselves.

Another object is that of providing biologically compatible prosthesis with improved resistance, duration and compatibility with the body that receives them.

Such object is attained through a method for the realization of a biologically compatible prosthesis component, characterized in that it comprises the steps of having at least two materials with different physical/chemical features, defining in forming means the component - which may be concave and/or convex and/or flat-shaped - as a composition of at least two volumes of said at least two materials, and sintering the component in said forming means and at a preset sintering temperature, according to what indicated in claim 1.

The use of two or more materials allows designing the component with less structural constraints, alongside allowing obtaining a porous surface for osteointegration on a base resistant to wear.

Preferably one or each of the two or more materials is initially available in form of powder, so as to best exploit the sintering techniques. In particular, a porous part may be obtained starting from a first powder material, possibly adding thereto a material called spacer, having a melting temperature that is greater than the sintering temperature of the first material. Subsequently, the spacer material is removed from the finished component hence creating corresponding empty spaces (pores) on the finished component. However, there are other techniques for forming the porous parts, see for example US 6 849 230. Thus, after sintering the piece may be entirely dense, given that the spacer is yet to be removed.

It may be provided for that one or each of these two or more materials may be a material initially available in sintered or pre-sintered form. This opens the field to numerous new products, which may exploit the physical/mechanical features of portions of the prosthesis realized or prepared before sintering. Same case applies should one or each of these two or more materials be a material already having a porous structure, which may be associated to the rest of the component sintered in the method described herein, but be produced previously, with the advantage of for example disregarding the parameters or requirements of the described sintering method.

Preferably, for production economy, the component is defined by overlapping the two or more materials in layers.

Preferably the sintering method is the SPS (Spark Plasma Sintering) method.

One or more of said materials may be made up of alloys of the same metal and/or different metals and/or pure metals.

The metals may be selected from among: Ti, Cr, Co, Mo, Fe, Zr, Nb, Ta, Mg.

The metal alloys may be selected from among groups that consist of: Cobalt-Chromium, Cobalt-Chromium-Molybdenum, Magnesium, Titanium-Aluminium, Titanium-Nickel, Zirconium-Niobium and Tantalum, Iron-Carbon and any combination therebetween.

Another variant is that one or each of said one or more materials is a ceramic material or a ceramic composite. The ceramic material may be selected from the group consisting of: Alumina, Zirconia, Zirconia stabilised with oxides such as for example Yttria, Ceria, Magnesia, etc., Zirconia Toughened Alumina (ZTA), Alumina Toughened Zirconia (ATZ), Chromium nitride, Silicon Carbide, Silicon Nitride, Titanium Carbide, Titanium Nitride, Zirconium Carbide, Zirconium Nitride, Tantalum Carbide, Tungsten Carbide, Hydroxyapatite, Calcium Phosphate, Magnesia, and any other combination therebetween.

In addition, one or each of said one or more materials may be a material made up of mixture of metal and/or ceramic powders or by a metal or ceramic or polymer matrix or a polymer matrix reinforced by fibrous or particle materials.

The invention also has the aim of providing a biocompatible prosthesis obtained according to the aforedescribed method.

### BRIEF DESCRIPTION OF THE DRAWINGS.

Further characteristics and advantages of the invention shall be clearer from the exemplifying description of the method, alongside the attached drawings wherein:
figure 1 shows an acetabulum cup producible through the invention;
figure 2 shows in vertical section a sintering press during a method according to the invention;
figure 3 shows an enlarged detail of fig 2,
figure 4 shows another object producible through the invention;
figure 5 shows a tibial plate producible through the invention.

### EMBODIMENTS OF THE INVENTION.

The present invention is suitable for obtaining orthopaedic prosthesis in general.

In general, the method is suitable for obtaining components, such as orthopaedic prosthesis and the like, made up of two or more materials 20, 22 having different physical/chemical characteristics, and which may be in different form in the finished piece depending on the specific application needs, for example dense, or porous, or a combination of both forms, as clarified hereinafter.

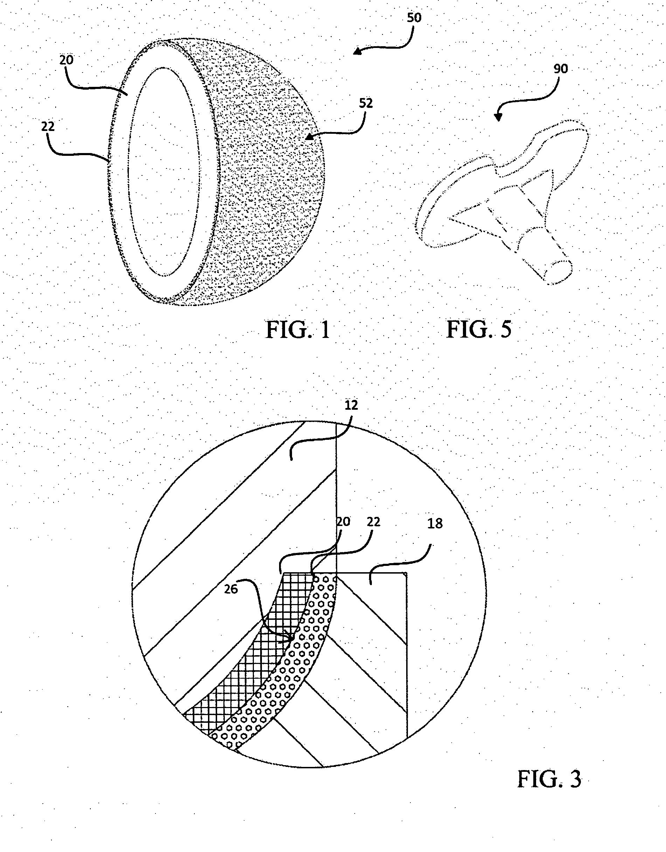

By way of non-limiting example, it is specified that the method described in an embodiment thereof is ideal for obtaining a hip prosthesis, for example an acetabulum cup 50 (fig 1). In such embodiment of the method according to the invention, the cup 50 obtained through the method itself has a porous convex external surface 52 (the one that shall end up in contact with the bone seat) made up of a first material 22 and an internal cup resistant to wear made of a second material 20 (dense part). The method of the invention may also be used for obtaining coating prosthesis, femoral stems, a tibial plate 90 (fig 5), for a knee prosthesis, unicompartimental prosthesis (and not for the knee), or generally orthopaedic prosthesis that consist of flat and/or curved geometries, with or without holes, fins, pins, etc.

More generally, it is specified that the present method may be applied to obtain objects, such as prosthesis and the like, of any shape, without any limitations whatsoever.

The materials 20, 22 preferably have different chemical/physical features and they are selected to maximise a different function with the properties thereof: maximum biointegration for the material 22, maximum resistance to wear and mechanical resistance for the material 20.

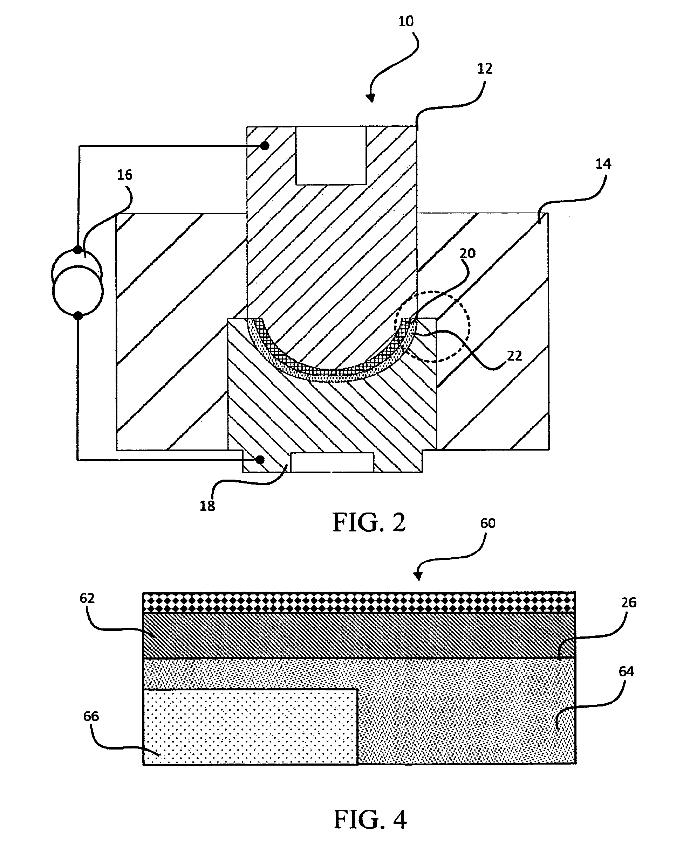

The cup 50 may be obtained through sintering methods that use pulsating currents such as for example the Spark Plasma Sintering (SPS), or, generally, through any sintering method (fig. 2, 3) which uses temperature and pressure simultaneously, for example using a given sintering temperature T1 suitably selected depending on the type of materials used. Sintering exploits a metal mould 10 made up of a body 14, a punch 12 and a die 18. Two overlapped layers of powder material 20, 22, possibly after mixing of the material 22 with a third removable spacer material 26, for example in form of granules, are arranged in the die 18.

The spacer material 26 has, suitably, a melting temperature T2 that is greater than the sintering temperature T1.

More in detail, the first material 22, intended for obtaining the porous part of the object to be obtained, is first mixed in a substantially homogeneous manner with the third spacer material 26, for example in form of granules, so as to obtain a mixture containing a spacer percentage equivalent to that of the porosity that is intended to be obtained on the final piece. The mixing step may be performed both manually and with more or less energetic mixing instruments, such as for example, ball milling, high energy milling, etc. The mixing of the first material 22 and the spacer 26 may also include the use of mixing additives such as water or volatile organic solvents which allow creating a slurry. The percentage in volume of the spacer material may generally range between 1% and 99% of the layer provided for. More preferably, the percentage in volume of the spacer material may generally vary between 10% and 80% of the layer provided for and even more preferably between 50% and 80%. The dimensions of the spacer material, for example in form of granules, may range between 5 and 3000 µm. More preferably the dimensions of the spacer material, for example in form of granules, may range between 100 and 1500 µm and even more preferably between 200 and 400 µm or between 400 and 600 µm or between 800 and 1000 µm.

Several layers of the same mixture with percentages of spacer material 26, for example, progressively decreasing, may be provided for to obtain a progressively decreasing porosity so as to provide greater support to the porous layer in the coupling thereof with the second material 20.

Subsequently, the first material 22, mixed with the third spacer material 26 according to the described methods, is arranged inside the die 18 in a manner such that the thickness of the layer is uniform over the entire profile of the die. This operation may be performed both manually and using instruments to facilitate the operation.

Then, a layer of the second material 20 is deposited on the layer of the first material 22. Two materials are considered in the example but the method may provide for even more, depending on the type and structure designed for the prosthesis to be obtained. Even the final structure may be considered as a multilayer, having for example an n number of materials for forming an n number of layers.

However, it should be observed that in other embodiments of the method according to the invention, the order of depositing the materials 20, 22 in the die 18 may be inverted, depending on the type of component to be obtained.

A voltage generator 16 is connected between the punch 12 and the die 18 with the aim of inducing a current between the two pieces. Such current also flows in the materials (conductors) 20, 22 and locally generates plasmas between the particles thereof, optimizing the densification of the powders. After completing sintering, during which the third spacer material 26 has not melted, the object made up of the three materials 20, 22, 26 is taken from the mould 10 and submerged in water or a suitable solution (acid or basic), so as to dissolve the material 26, remove it from the host material 22 leaving it partly porous. The space emptied by the material 26 constitutes the empty spaces or pores of the porous part 52.

An embodiment of the method according to the invention comprises, at this point, a further step of optimising the densification of the component obtained through thermal treatments, performed at a temperature lower than the lowest among the melting temperatures of the materials that constitute the component, with or without using a given treatment pressure (for example methods such as Hot Isostatic Pressing).

Another embodiment of the method according to the invention comprises, at this point, a further step of performing mechanical post-machining operations on the obtained component to modify the surface quality of a part or the entire component, for example in terms of surface roughness.

Another embodiment of the method according to the invention comprises, at this point, a further step of depositing, on a part of the component or on the entire component, a single or multi-layer coating, through the Plasma Spray technique, a biologically compatible material which helps the osteointegration of Titanium and/or Hydroxyapatite and/or a material belonging to the class of Calcium-Phosphates.

Another embodiment of the method according to the invention comprises, at this point, a further step of depositing, on a part of the component or on the entire component, a single or multi-layer coating, through chemical or electrochemical means, a biologically compatible material which helps the osteointegration of Hydroxyapatite and/or a material belonging to the class of Calcium-Phosphates.

Another embodiment of the method according to the invention comprises, at this point, a further step of depositing, on a part of the component or on the entire component, a single or multi-layer coating, through the Physical Vapor Deposition (PVD) or Chemical Vapor Deposition (CVD) techniques, of a biologically compatible material capable of facilitating osteointegration.

Another embodiment of the method according to the invention comprises, at this point, at least one further step of performing surface treatments on a part or on the entire component suitable to modify the surface roughness of the same components. Examples of such treatments may be sanding, or other treatments both mechanical and chemical.

Depending on the type and number of materials and layers present in the sintering method, the final components provided according to the invention may for example be made up of or comprise:
- different materials for the dense and porous part which differ due to the fact that they are in pure or alloy form (for example Ti grade 5 for the dense part and Ti grade 1-4 for the porous part);
- two or more alloys of the same metal (for example Ti6Al4V, Ti-Ni, Ti6Al7Nb, Ti13Nb13Zr, Ti12Mb6Al, alloys of Ta);
- two or more different materials: preferably Titanium for the porous part and an alloy of CoCrMo for the dense part;
- a multilayer material made up of layers of Titanium grade 2 (porous layer, for example with MgO to be removed), Titanium grade 1-4 or 5 (dense layer) and CoCr (dense layer). In this type of structures, the dense titanium layer may serve as an barrier for preventing the ionic release of CoCr in the body, a problem unsolved over a very long period of time;
- one or more materials in powder form 62, 64 (see object 60 in fig 4), deposited as a layer or in a volume in any form, and one or more composite or ceramic powder materials 66, deposited as a layer or in a volume of any form. The composite material, in turn, may be made up of a matrix and reinforcement particles or fibres.
- the ceramic material is selected from among the group comprising: Alumina, Zirconia, Zirconia stabilised with oxides such as for example Yttria, Ceria, Magnesia, etc, Zirconia Toughed Alumina (ZTA), Alumina Toughened Zirconia (ATZ), Chromium nitride, Silicon Carbide, Silicon Nitride, Titanium Carbide, Titanium Nitride, Zirconiun Carbide, Zirconium Nitride, Tantalum Carbide, Tungsten Carbide, Hydroxyapatite, Calcium-phosphates, Magnesia, and every combination therebetween.
   The following may be used as ceramic materials for such purpose: Zirconium Oxide (Zirconia), Y-TZP (Yttria Stabilised Zirconia), PSZ (Zirconia stabilized for example using MgO, CeO2, etc), Alumina, ZTA (Zirconia Toughened Alumina), ATZ (Alumina Toughened Zirconia). The matrix may contain carbon fibres;
- a layer of polymer material (e.g. PE or UHMWPE or PEEK);
- two or more porous portions coupled to each other, obtained for example through selective removal of spacer material from two or more layers of different material or sintering two or more previously sintered porous materials therebetween;
- a grid or network porous material obtained previously (for example sintered previously) which is coupled to a powder material which is sintered through the described method.

MgO may be used as the spacer material 26 but even for example sodium chloride or hydroxyapatite may also be used.

The sintering temperatures and the other parameters of the method depend on many variables, among which the type of object to be produced, the shape thereof, the constituting materials and the materials with which the mould is made. For example, should one want to obtain a prosthesis component having a Co-Cr alloy as a constituent of the dense part and Titanium grade 4 as the constituent of the porous part, the sintering temperature may range between 500-2000°C, the pressure may range between 5-100 MPa and the duration of the sintering may vary between 1-20 minutes.

These values, just like for the currents circulating in the mould, should be optimized from time to time. They strongly depend on the materials, the shape of the mould and the geometry of the object to be sintered.

Regarding, in particular, objects made using the first material 22 and the second material 20 of different type and thus with different characteristics, it is hereby also pointed out the following.

Should the two or more materials 20, 22 have a different coefficient of thermal dilation, as it for example occurs in cases whereby the second material 20 is a material of the ceramic type of materials, while the first material 22 is a metal material, at the end of the sintering method, the cooling of the produced piece determines a considerable contraction of the first metal material 22, such contraction instead being lower in the second material 20, considerably lower or absent in case of ceramic material.

This effect, particularly but not exclusively if the produced piece has a substantially acetabular form or the like, but also in the substantially flat pieces, allows obtaining a better quality coupling between the two materials 20, 22, for example better than the traditional conical coupling used in this type of objects. Actually, it is known in the field that the interface area between the two materials 20,2 2 constitutes a weak link for this type of objects: a conventional conical coupling, for example, may detach easily and inadvertently.

It should thus be observed that the contraction of the first metal material 22 determines the occurrence of a given internal "lock" tension between the two materials 20, 22, which in particular withholds the second material 20 within the first material 22 in a considerably firm, rigid and precise manner with respect to what occurs in the pieces produced through conventional methods. Furthermore, due to this effect, thinner metal layers may be obtained, increasing the thickness of the ceramic layer.

Another embodiment of the method according to the invention provides for the realization of a component made up of two materials 20, 22, with different physical/chemical features, which define two respective volumes both dense, i.e. without any porous area, so as to meet the needs of some specific applications which require this solution, for example applications which provide for a subsequent step of creating a porous coating through the Plasma Spray technique or the like.

The method, thus comprises at least the steps of defining the component, in the forming means 10, as a composition of two volumes of different materials 20, 22, followed by a step of sintering, in the forming means 10, the two volumes of materials 20, 22, which in particular acquire the form, in the obtained component, of two respective adjacent or coupled layers.

In the case of association between ceramic and metal material, the previously described improvement of coupling quality effects may be obtained.

At this point, possible further steps, such as surface treatments, mechanical machinings, deposition of coating layers (for example through Plasma Spray), etc may be carried out as described previously to obtain a component having the desired characteristics.

In another embodiment of the method according to the invention, the component is defined, in the forming means 10, as the composition of volumes of materials 20, 22 of different physical/chemical features, and at least one volume of a third spacer material 26.

More in detail, the component to be obtained is defined as a succession of volumes of the above-mentioned materials 20,22,26. For example, it is possible to define, in the forming means 10, the component 50,60,90 as made up of at least one layer of the second material 20, coupled to at least one layer of the first material 22, to which at least one layer of the third spacer material 26, for example in form of granules, however not mixed to any other material in this case is associated in turn (the order of the layers may obviously be inverted depending on the type of component to be obtained).

Thus, at the end of the sintering step provided for, the third spacer material 26 is removed, through the aforementioned techniques, so as to create a rough surface on the component 50,60,90. The roughness of the surface may be defined, for example, by the size of the granules of the third spacer material 26.

At the end of the latter step, further steps, such as surface treatment, mechanical machinings, deposition of coating layers, etc may be performed as described previously to obtain a component of the desired characteristics.

## Claims

1. Process for the realization of a biologically compatible prosthesis component (50;60;90), **characterized by** comprising the steps of
- providing at least two materials (20, 22, 26) with different physical/chemical features;
- defining in forming means (10) the component (50;60;90) as a composition of at least two volumes of said at least two materials (20,22,26), wherein one or each of said at least two materials (20,22,26) is initially available in form of powder and wherein said volumes of said materials (20, 22, 26) are made by adjoining layers;
- sintering the component (50,60,90) in said forming means (10) at a certain sintering temperature (T1) through the Spark Plasma sintering (SPS) that uses pulsating currents or through any sintering method that uses temperature and pressure simultaneously and that uses pulsating currents, wherein a voltage generator (16) is connected between a punch (12) and a die (18) of said forming means (10) inducing a current between said materials (20,22), wherein said current flows in said materials (20,22) and locally generates plasmas between the particles thereof, optimizing the densification of said powders and obtaining said layers of said materials (20,22) that are dense.

2. Process according to claim 1, in which said layers of said materials (20,22) comprise a first porous layer of said first material (22) and a second dense layer of said second material (20), wherein said porous layer is obtained by adding, to said first material (22), a third spacer material (26), in the powder form and having a melting temperature (T2) higher than said sintering temperature (T1) and by removing from said finished component (50,60,90) said third spacer material (26) to leave on said finished component (50,60,90) corresponding void spaces.

3. Process according to claim 1 or 2, in which said layers of said materials (20,22) comprise a rough layer of said first material (22) and a dense layer of said second material (20).

4. Process according to claim 3, comprising a step of depositing, on the layer of said first material (22), at least a layer of said third spacer material (26).

5. Process according to claim 4, comprising a step of removing, from said finished component (50,60,90), said third spacer material (26) to leave on said finished component (50,60,90) a corresponding rough surface.

6. Process according to any one of the preceding claims, wherein both said materials (20,22) are in powder form.

7. Process according to claim 1, wherein said second material (20) is an already sintered material or a pre-sintered material.

8. Process according to any one of the preceding claims, wherein one or more of said materials (20,22) consists of alloys of the same metal and/or of different metals and/or of pure metals.

9. Process according to claim 8, wherein the metal is chosen from the group comprising Ti, Cr, Co, Mo, Fe, Zr, Mn, Ta, Mg and/or metal alloys selected from the group comprising: Cobalt-Chromium, Chromium-Cobalt- Molibdenum, Magnesium, Titanium-Aluminum, Zirconium-Niobium and Tantalum, Iron-Carbon, and any combination of them.

10. Process according to any one of the preceding claims, wherein one or each of said one or more materials (20,22) is a ceramic material or a ceramic composite.

11. Process according to claim 10, in which the ceramic material is selected from the group comprising: Alumina, Zirconia, Zirconia stabilised with oxides such as for example Yttria, Ceria, Magnesia, etc., Zirconia Toughened Alumina (ZTA), Alumina Toughened Zirconia (ATZ), Chromium Nitride, Silicon Carbide, Silicon Nitride, Titanium Carbide, Titanium Nitride, Zirconium Carbide, Zirconium Nitride, Tantalum Carbide, Tungsten Carbide, Hydroxyapatite, Calcium Phosphate, Magnesia, and any other combination therebetween.

12. Process according to any one of the preceding claims, in which one or each of said one or more materials (20,22) is a material formed by mixtures of metal and/or ceramic powders or by a metal or ceramic or polymer matrix or a polymer matrix reinforced by fibrous or particulate materials.

13. Process according any one of the preceding claims, comprising a step of performing mechanical post-machining operations and/or surface treatments on the component (50;60;90) realized to modify the surface quality of a part or of the whole component (50;60;90).

14. Process according to claim 13, comprising a step of depositing, on a part of the component or on the entire component (50;60;90), a single or multi-layer coating, through the Physical Vapor Deposition (PVD) or Chemical Vapor Deposition (CVD) technique or Plasma Spray o through chemical and/or electrochemical means, of a biologically compatible material capable of facilitating osteointegration.

## Patentansprüche

1. Verfahren zur Herstellung einer biologisch verträglichen Prothesenkomponente (50; 60; 90), **dadurch gekennzeichnet, dass** es die folgenden Schritte umfasst:
- Bereitstellen von mindestens zwei Materialien (20, 22, 26) mit verschiedenen physikalischen/chemischen Eigenschaften;
- Definieren in Formmitteln (10) der Komponente (50; 60; 90) als eine Anordnung von mindestens zwei Volumen der mindestens zwei Materialien (20, 22, 26), wobei eines oder jedes der mindestens zwei Materialien (20, 22, 26) anfangs in Pulverform vorhanden ist und wobei diese Volumen dieser Materialien (20, 22, 26) aus angrenzenden Schichten bestehen;
- Sintern der Komponente (50, 60, 90) in den Formmitteln (10) bei einer bestimmten Sintertemperatur (T1) durch das Spark Plasma Sintern (SPS), das gepulste Ströme verwendet, oder durch ein beliebiges Sinterverfahren, das gleichzeitig Temperatur und Druck verwendet und das gepulste Ströme verwendet, wobei ein Spannungsgenerator (16) zwischen einen Stempel (12) und eine Matrize (18) der Formmittel (10) geschaltet ist und einen Strom zwischen den Materialien (20, 22) induziert, wobei dieser Strom in diesen Materialien (20, 22) fließt und örtlich Plasmen zwischen deren Partikeln erzeugt, wodurch die Verdichtung der Pulver optimiert wird und die Schichten dieser Materialien (20,22) erhalten werden, die dicht sind.

2. Verfahren nach Anspruch 1, wobei die Schichten der Materialien (20, 22) eine erste poröse Schicht des ersten Materials (22) und eine zweite dichte Schicht des zweiten Materials (20) umfassen, wobei die poröse Schicht durch Hinzufügen zum ersten Material (22) eines dritten Platzhaltermaterials (26), das pulverförmig ist und eine Schmelztemperatur (T2) hat, die höher als die Sintertemperatur (T1) ist, und durch Entfernen dieses dritten Platzhaltermaterials (26) von der fertigen Komponente (50, 60, 90), um auf der fertigen Komponente (50, 60, 90) entsprechende Hohlräume zu lassen, erhalten wird.

3. Verfahren nach Anspruch 1 oder 2, wobei die Schichten der Materialien (20, 22) eine raue Schicht des ersten Materials (22) und eine dichte Schicht des zweiten Materials (20) umfassen.

4. Verfahren nach Anspruch 3, das einen Schritt des Aufbringens von mindestens einer Schicht des dritten Platzhaltermaterials (26) auf die Schicht des ersten Materials (22) umfasst.

5. Verfahren nach Anspruch 4, das einen Schritt des Entfernens des dritten Platzhaltermaterials (26) von der fertigen Komponente (50, 60, 90) umfasst, um auf der fertigen Komponente (50, 60, 90) eine entsprechende raue Oberfläche zu lassen.

6. Verfahren nach einem der vorhergehenden Ansprüche, wobei die beiden Materialien (20, 22) pulverförmig sind.

7. Verfahren nach Anspruch 1, wobei das zweite Material (20) ein schon gesintertes Material oder ein vorgesintertes Material ist.

8. Verfahren nach einem der vorhergehenden Ansprüche, wobei eines oder mehrere der Materialien (20, 22) aus Legierungen desselben Metalls und/oder von verschiedenen Metallen und/oder aus reinen Metallen bestehen.

9. Verfahren nach Anspruch 8, wobei das Metall aus der Gruppe ausgewählt ist, die Ti, Cr, Co, Mo, Fe, Zr, Mn, Ta, Mg und/oder Metalllegierungen umfasst, die aus der Gruppe ausgewählt sind, die Folgendes umfasst: Cobalt-Chrom, Chrom-Cobalt-Molybdän, Magnesium, Titan-Aluminium, Zirkonium-Niobium und Tantal, Eisen-Kohlenstoff und eine beliebige Kombination davon.

10. Verfahren nach einem der vorhergehenden Ansprüche, wobei eines oder jedes des einen oder der mehreren Materialien (20, 22) ein Keramikmaterial oder ein Keramikverbundwerkstoff ist.

11. Verfahren nach Anspruch 10, wobei das Keramikmaterial aus der Gruppe ausgewählt ist, die Folgendes umfasst: Aluminiumoxid, Zirconiumdioxid, mit Oxiden wie beispielsweise Yttriumoxid, Cerdioxid, Magnesiumoxid usw. stabilisiertes Zirconiumdioxid, mit Zirconiumdioxid verstärktes Aluminiumoxid (ZTA), mit Aluminiumoxid verstärktes Zirconiumdioxid (ATZ), Chrom(III)-nitrid, Siliciumcarbid, Siliciumnitrid, Titancarbid, Titannitrid, Zirconiumcarbid, Zirconiumnitrid, Tantalcarbid, Wolframcarbid, Hydroxylapatit, Calciumphosphat, Magnesia und jede beliebige andere Kombination zwischen diesen.

12. Verfahren nach einem der vorhergehenden Ansprüche, wobei eines oder jedes des einen oder der mehreren Materialien (20, 22) ein Material ist, das aus Gemischen von Metall- und/oder Keramikpulvern oder aus einer Metall- oder Keramik- oder Polymermatrix oder aus einer mit faser- oder teilchenförmigen Materialien verstärkten Polymermatrix gebildet ist.

13. Verfahren nach einem der vorhergehenden Ansprüchen, das einen Schritt des Ausführens von mechanischen Nachbearbeitungsvorgängen und/oder Oberflächenbehandlungen an der Komponente (50; 60; 90) umfasst, die durchgeführt werden, um die Oberflächenqualität eines Teils oder der ganzen Komponente (50; 60; 90) zu verändern.

14. Verfahren nach Anspruch 13, das einen Schritt des Aufbringens einer ein- oder mehrlagigen Beschichtung aus einem biologisch verträglichen Material, das imstande ist, die Osseointegration zu begünstigen, auf einen Teil der Komponente oder auf die gesamte Komponente (50; 60; 90) durch das Verfahren zur physikalischen Gasphasenabscheidung (PVD) oder zur chemischen Gasphasenabscheidung (CVD) oder durch Plasmaspritzen oder durch chemische und/oder elektrochemische Mittel umfasst.

## Revendications

1. Procédé pour la réalisation d'un composant de prothèse biologiquement compatible (50 ; 60 ; 90), **caractérisé en ce qu'**il comprend les étapes suivantes :
- la fourniture d'au moins deux matériaux (20, 22, 26) avec des caractéristiques physiques/chimiques différentes ;
- la définition dans des moyens de formage (10) du composant (50 ; 60 ; 90) comme une composition d'au moins deux volumes desdits au moins deux matériaux (20, 22, 26), dans lequel un ou chacun desdits au moins deux matériaux (20, 22, 26) est initialement disponible sous forme de poudre et dans lequel lesdits volumes desdits matériaux (20, 22, 26) sont réalisés en jouxtant des couches ;
- le frittage du composant (50, 60, 90) dans lesdits moyens de formage (10) à une certaine température de frittage (T1) par le frittage par plasma à étincelle (SPS) qui utilise des courants pulsés ou par un quelconque procédé de frittage qui utilise simultanément la température et la pression et qui utilise des courants pulsés, dans lequel un générateur de tension (16) est connecté entre un poinçon (12) et une matrice (18) desdits moyens de formage (10) induisant un courant entre lesdits matériaux (20, 22), dans lequel ledit courant circule dans lesdits matériaux (20, 22) et génère localement des plasmas entre leurs particules, en optimisant la densification desdites poudres et en obtenant lesdites couches desdits matériaux (20, 22) qui sont denses.

2. Procédé selon la revendication 1, dans lequel lesdites couches desdits matériaux (20, 22) comprennent une première couche poreuse dudit premier matériau (22) et une deuxième couche dense dudit deuxième matériau (20), dans lequel ladite couche poreuse est obtenue en ajoutant, audit premier matériau (22), un troisième matériau d'écartement (26), sous la forme de poudre et ayant une température de fusion (T2) supérieure à ladite température de frittage (T1), et en enlevant dudit composant fini (50, 60, 90) ledit troisième matériau d'écartement (26) pour laisser sur ledit composant fini (50, 60, 90) des espaces vides correspondants.

3. Procédé selon la revendication 1 ou 2, dans lequel lesdites couches desdits matériaux (20, 22) comprennent une couche rugueuse dudit premier matériau (22) et une couche dense dudit deuxième matériau (20).

4. Procédé selon la revendication 3, comprenant une étape de dépôt, sur la couche dudit premier matériau (22), d'au moins une couche dudit troisième matériau d'écartement (26).

5. Procédé selon la revendication 4, comprenant une étape consistant à enlever, dudit composant fini (50, 60, 90), ledit troisième matériau d'écartement (26) pour laisser sur ledit composant fini (50, 60, 90) une surface rugueuse correspondante.

6. Procédé selon l'une quelconque des revendications précédentes, dans lequel lesdits matériaux (20, 22) sont tous deux sous forme de poudre.

7. Procédé selon la revendication 1, dans lequel ledit deuxième matériau (20) est un matériau déjà fritté ou un matériau pré-fritté.

8. Procédé selon l'une quelconque des revendications précédentes, dans lequel un ou plusieurs desdits matériaux (20, 22) sont constitués d'alliages du même métal et/ou de métaux différents et/ou de métaux purs.

9. Procédé selon la revendication 8, dans lequel le métal est choisi à partir du groupe comprenant Ti, Cr, Co, Mo, Fe, Zr, Mn, Ta, Mg et/ou des alliages métalliques sélectionnés à partir du groupe comprenant : cobalt-chrome, chrome-cobalt-molybdène, magnésium, titan-aluminium, zirconium-niobium et tantale, fer-carbone et n'importe quelle combinaison de ceux-ci.

10. Procédé selon l'une quelconque des revendications précédentes, dans lequel un ou chacun desdits un ou plusieurs matériaux (20, 22) est un matériau céramique ou un composite céramique.

11. Procédé selon la revendication 10, dans lequel le matériau céramique est sélectionné à partir du groupe comprenant : alumine, zircone, zircone stabilisé avec des oxydes tels que, par exemple, oxyde d'yttrium, oxyde de cérium, magnésie, etc., alumine renforcée en zircone (ZTA), zircone renforcé en alumine (ATZ), nitrure de chrome, carbure de silicium, nitrure de silicium, carbure de titane, nitrure de titane, carbure de zirconium, nitrure de zirconium, carbure de tantale, carbure de tungstène, hydroxyapatite, phosphate de calcium, magnésie, et n'importe quelle combinaison entre eux.

12. Procédé selon l'une quelconque des revendications précédentes, dans lequel un ou chacun desdits un ou plusieurs matériaux (20, 22) est un matériau formé par des mélanges de poudres métalliques et/ou céramiques ou par une matrice métallique ou céramique ou polymère ou une matrice polymère renforcée par des matériaux fibreux ou particulaires.

13. Procédé selon l'une quelconque des revendications précédentes, comprenant une étape d'exécution d'opérations post-usinage mécanique et/ou de traitements de surface sur le composant (50 ; 60 ; 90) réalisées pour modifier la qualité de surface d'une partie ou de la totalité du composant (50; 60; 90).

14. Procédé selon la revendication 13, comprenant une étape de dépôt, sur une partie du composant ou sur la totalité du composant (50; 60; 90), d'un revêtement monocouche ou multicouche, par le biais de la technique de dépôt physique en phase vapeur (PVD) ou de dépôt chimique en phase vapeur (CVD) ou de projection de plasma ou par le biais de moyens chimiques et/ou électrochimiques, d'un matériau biologiquement compatible capable de faciliter l'ostéointégration.
